(19) **Europäisches Patentamt**
**European Patent Office**
**Office européen des brevets**

(11) **EP 1 473 058 B1**

(12) **FASCICULE DE BREVET EUROPEEN**

(45) Date de publication et mention
de la délivrance du brevet:
**04.06.2008 Bulletin 2008/23**

(51) Int Cl.:
*A61N 7/00* (2006.01)        *A61N 1/30* (2006.01)
*A61H 1/00* (2006.01)

(21) Numéro de dépôt: **04290140.5**

(22) Date de dépôt: **20.01.2004**

(54) **Dispositif d'analyse de la peau comportant une sonde ultrasonore**

Vorrichtung zur Analyse der Haut mit einer Ultraschallprobe

Apparatus for analysis of the skin comprising an ultrasonic probe

(84) Etats contractants désignés:
**AT BE BG CH CY CZ DE DK EE ES FI FR GB GR HU IE IT LI LU MC NL PT RO SE SI SK TR**

(30) Priorité: **23.01.2003 FR 0300721**

(43) Date de publication de la demande:
**03.11.2004 Bulletin 2004/45**

(73) Titulaire: **L'ORÉAL**
**75008 Paris (FR)**

(72) Inventeurs:
• **Querleux, Bernard**
**94170 Le Perreux (FR)**

• **Baldeweck, Thérèse**
**94300 Vincennes (FR)**
• **Fink, Mathias**
**92190 Meudon (FR)**

(74) Mandataire: **Tanty, François et al**
**Nony & Associés,**
**3, rue de Penthièvre**
**75008 Paris (FR)**

(56) Documents cités:
**FR-A- 2 791 136          FR-A- 2 811 524**
**US-B1- 6 273 864**

**Description**

La présente invention concerne les procédés et dispositifs d'analyse de la peau humaine.

L'invention concerne plus particulièrement mais non exclusivement un dispositif dans lequel une information utile concernant au moins une propriété du tissu est obtenue en analysant à l'aide d'une sonde ultrasonore la propagation d'une onde de cisaillement dans celui-ci. Un tel dispositif est décrit dans la demande internationale WO 00/55616.

Le document FR 2655835 décrit un appareil de détection de kystes.

La présente invention vise à proposer un dispositif permettant une analyse de la peau sur une faible profondeur à partir de sa surface, afin par exemple de déterminer les modules d'Young et de cisaillement des différentes couches constitutives du derme et de l'hypoderme ainsi que de déterminer leur épaisseur, le cas échéant.

L'invention a ainsi pour objet, selon l'un de ses aspects parmi d'autres, un dispositif d'analyse de la peau qui peut se caractériser par le fait qu'il comporte :

- une sonde ultrasonore agencée pour analyser la peau selon un axe X,
- un vibreur agencé pour transmettre à une région de la peau s'étendant autour de l'axe X au moins une onde de cisaillement, la sonde ultrasonore étant agencée pour détecter des déplacements induits dans la peau par la propagation de l'onde de cisaillement.

Le fait que l'onde de cisaillement soit générée dans la peau à partir d'une région s'étendant autour de l'axe X peut permettre d'accroître l'amplitude des déplacements de la peau selon l'axe X, et donc d'accroître la précision de la mesure. Le dispositif peut comporter une sonde ultrasonore unique, et malgré l'utilisation d'une seule sonde, la précision obtenue peut s'avérer satisfaisante.

Le dispositif comporte avantageusement un organe de couplage permettant la transmission d'ondes ultrasonores entre la sonde et la peau. La sonde ayant une distance focale, l'épaisseur de l'organe de couplage est choisie de préférence de manière à permettre la focalisation des ondes ultrasonores dans une région de profondeur maximale donnée à partir de la surface de la peau. Cette profondeur peut être inférieure ou égale à 4 mm par exemple, afin de permettre une analyse des propriétés mécaniques du derme et de l'hypoderme de la peau.

La distance focale de la sonde ultrasonore peut être comprise par exemple entre 10,4 mm et 15,6 mm, étant par exemple égale à 13 mm environ dans un exemple particulier de mise en oeuvre de l'invention.

L'épaisseur de l'organe de couplage peut être comprise par exemple entre 10,6 mm et 14,4 mm, étant par exemple égale à 12 mm environ dans le cas où la distance focale précitée est de 13 mm environ.

L'organe de couplage peut comporter une couche d'un matériau viscoélastique présentant un module d'Young proche de celui de la peau, par exemple un module d'Young compris entre 0,25 et 25 MPa, par exemple 0,5 et 25 MPa, notamment de l'ordre de 0,5 MPa. Cette couche de matériau viscoélastique peut par exemple être une couche d'un gel, notamment un gel comportant de la gélatine à une concentration comprise entre 5 et 25 %, notamment de 10 % environ.

Le matériau viscoélastique de l'organe de couplage peut se présenter par exemple sous la forme d'un disque, notamment d'un disque d'épaisseur sensiblement constante, ce disque pouvant être maintenu contre la surface de la peau par un anneau de maintien pourvu d'un retour vers l'intérieur, contre lequel la face du disque opposée à la peau peut prendre appui. Cet anneau peut faire partie d'un châssis dont sont solidaires le vibreur et la sonde, ce châssis étant de préférence réalisé de manière à permettre de positionner le dispositif à la surface de la peau de telle manière que l'axe X soit sensiblement perpendiculaire à la surface de la peau.

Le vibreur peut comporter une pièce annulaire définissant une surface de contact à partir de laquelle l'onde de cisaillement est émise vers la peau, cette pièce annulaire présentant une lumière centrale dans laquelle s'étend la sonde ultrasonore.

La surface de contact peut présenter une symétrie par rapport à l'axe X et notamment une symétrie de révolution autour de l'axe X. Ainsi, l'onde de cisaillement peut être transmise dans la peau à partir d'une excitation axisymétrique autour de l'axe X.

La sonde peut être agencée pour transmettre et recevoir les ondes ultrasonores à une fréquence comprise entre 1 et 300 MHz par exemple, mieux entre 10 et 300 MHz, voire entre 30 et 70 MHz, mieux encore supérieure à 40 MHz, par exemple de 50 MHz environ. Une telle fréquence permet d'obtenir une résolution axiale satisfaisante, par exemple meilleure que 100 $\mu$m. Le vibreur peut comporter un dispositif électromagnétique comportant par exemple une bobine et le dispositif d'analyse peut comporter un générateur agencé pour alimenter le vibreur avec un signal basse fréquence pendant toute la durée de l'analyse, par exemple un signal de fréquence comprise entre 100 et 500 Hz, de 300 Hz environ dans un exemple particulier de mise en oeuvre de l'invention. Un tel signal s'amortit rapidement dans la peau humaine et peut permettre de ne pas générer trop de perturbations dues à des échos. Le vibreur peut encore comporter au moins un organe pneumatique ou hydraulique, l'onde de cisaillement étant dans ce cas générée à partir d'une variation de pression d'un gaz ou d'un liquide.

Le dispositif d'analyse peut comporter un dispositif de traitement agencé pour délivrer au moins une information représentative d'une propriété mécanique d'une couche au moins de la peau, à partir des signaux recueillis par la sonde ultrasonore. Cette information peut par exemple comporter la valeur du module d'Young et/ou du module de cisaillement et/ou de l'épaisseur du derme ou de l'hypoderme.

Le dispositif de traitement peut être agencé pour délivrer une information relative à l'état de la peau, par exemple son état de vieillissement, à partir de la comparaison de la valeur mesurée avec des valeurs de référence.

Le dispositif de traitement peut être agencé pour effectuer une mémorisation des signaux recueillis par la sonde ultrasonore à différents instants successifs, par exemple à des intervalles de temps *dt* pendant *n* fois, *dt* étant compris par exemple entre 0,2 et 0,8 ms et *n* compris entre 50 et 500, et effectuer un traitement statistique des signaux recueillis afin d'améliorer le rapport signal/bruit.

Le traitement statistique peut par exemple comporter le calcul d'une valeur moyenne pour le module d'Young E ou le module de cisaillement $\mu$ ou la vitesse de propagation Vs de l'onde de cisaillement.

Avantageusement, la sonde et le vibreur sont agencés de telle sorte que le déplacement du vibreur, selon l'axe X, permettant de générer l'onde de cisaillement n'est pas transmis à la sonde.

Dans une réalisation particulière, le vibreur transmet l'onde de cisaillement à la peau à travers l'organe de couplage. L'organe de couplage peut alors avantageusement contribuer à faire en sorte que l'onde de cisaillement qui atteint la région d'analyse présente une forme adaptée à la mesure, étant alors notamment suffisamment éloignée du lieu où elle a pris naissance.

Le dispositif peut être agencé de manière à ce que l'un au moins de la sonde ultrasonore et du vibreur soit relié à un dispositif de traitement tel qu'un micro-ordinateur, mais on ne sort pas du cadre de la présente invention lorsque la sonde, le vibreur et le dispositif de traitement sont intégrés au sein d'un appareil portable, tenant dans une seule main par exemple, comportant une face d'application sur la peau, et par exemple au moins un afficheur permettant de délivrer une information liée à la région analysée.

Le vibreur peut encore comporter au moins une buse permettant de diriger sur la surface de la peau ou d'un organe de couplage un jet d'un liquide ou d'un gaz comprimé, par exemple de l'air comprimé.

Le vibreur peut encore comporter des moyens permettant de générer l'onde de cisaillement grâce à une dépression exercée localement sur la peau ou l'organe de couplage.

Le cas échéant, le dispositif et notamment la partie d'acquisition destinée à être au contact de la peau peut comporter un ou plusieurs capteurs destinés par exemple à mesurer l'hydratation, le micro-relief de la peau, le pH, la température ou la couleur, l'humidité à la surface du tissu, voire au moins un biocapteur.

L'invention a encore pour objet, selon un autre de ses aspects, en combinaison ou indépendamment de ce qui précède, un dispositif d'analyse de la peau, qui peut se caractériser par le fait qu'il comporte :

- une sonde ultrasonore pour analyser la peau selon un axe X,
- un vibreur agencé pour transmettre à une région de la peau s'étendant le long de l'axe au moins une onde de cisaillement, la sonde ultrasonore étant capable de détecter des déplacements induits dans la peau par la propagation de l'onde de cisaillement,
- un organe de couplage, cet organe de couplage étant choisi de manière à permettre la focalisation des ondes ultrasonores émises par la sonde ultrasonore dans une région ayant une profondeur maximale donnée à partir de la surface de la peau, cette profondeur étant par exemple inférieure ou égale à 4 mm.

L'invention a encore pour objet, selon un autre de ses aspects, un procédé d'analyse de la peau qui peut se caractériser par le fait qu'il comporte l'étape consistant à analyser la peau au moyen d'un dispositif tel que l'un de ceux définis plus haut.

Dans une mise en oeuvre particulière de l'invention, le procédé peut comporter en outre l'étape de traitement des signaux provenant de la sonde ultrasonore afin de déterminer au moins une valeur liée à des propriétés mécaniques de la peau, notamment le module d'Young, le module de cisaillement $\mu$ ou la vitesse de propagation Vs de l'onde de cisaillement.

Le procédé peut encore comporter un traitement des signaux provenant de la sonde ultrasonore afin de déterminer l'épaisseur du derme ou de l'hypoderme.

Le traitement des signaux peut comporter une étape de calcul du retard de phase de l'onde de cisaillement en fonction de la profondeur.

Le traitement des signaux peut comporter un procédé de traitement par intercorrélation, tel que décrit dans la demande internationale WO 00/55616 précitée.

Le traitement peut viser à déterminer un état de la peau, notamment un état de vieillissement de celle-ci, à partir par exemple de la comparaison d'une valeur de module d'Young déterminée par l'analyse de la peau avec des valeurs de référence, exploitant ainsi le fait que la vitesse de propagation de l'onde de cisaillement dans le derme et le module d'Young tendent à être plus faibles chez les personnes jeunes que chez les personnes âgées.

Le traitement peut encore viser à déterminer la tension de la peau, sachant que la vitesse de propagation de l'onde de cisaillement et le module d'Young dans un milieu tendu sont plus élevés que dans un milieu relâché.

L'invention a encore pour objet, selon un autre de ses aspects, un procédé pour évaluer la peau, comportant les étapes suivantes :

- analyser la peau au moyen d'un dispositif tel que défini plus haut,

- délivrer à partir des résultats de l'analyse une information relative à une propriété mécanique au moins de ladite région, par exemple son élasticité.

Le cas échéant, on peut délivrer une information relative à l'anisotropie des fibres de collagène dans le plan de la peau, notamment si l'onde de cisaillement est transmise dans la peau par une surface de contact du vibreur présentant une géométrie non axisymétrique, cette surface de contact étant par exemple définie par un barreau, au lieu d'une pièce annulaire.

On peut comparer les résultats de deux évaluations de la peau, notamment les valeurs de module d'Young, à deux instants différents, et délivrer une information relative à l'évolution d'une propriété mécanique au moins de la peau, notamment son élasticité, entre ces deux instants. Cela peut permettre, par exemple, de renseigner l'individu objet des évaluations sur l'incidence d'un traitement.

On peut aussi effectuer au moins une évaluation des propriétés mécaniques, notamment l'élasticité, d'une région du corps non exposée à un environnement donné, par exemple non exposée au soleil, et au moins une évaluation d'une région du corps exposée à cet environnement et comparer les résultats, et déterminer à partir de la comparaison de ces résultats une information utile pour évaluer le vieillissement de la peau, par exemple.

Par ailleurs, on peut analyser le tissu avec le capteur en un premier emplacement géographique, par exemple dans un institut de beauté, sur un point de vente ou à domicile, et transmettre à distance, par un réseau Internet, Intranet ou de téléphonie mobile, des données obtenues avec le dispositif, et traiter à un deuxième emplacement géographique, par exemple dans un centre de recherche, lesdites données, de manière à évaluer une propriété de la peau, son élasticité par exemple.

On peut transmettre le résultat de l'évaluation par un réseau Internet, Intranet ou de téléphonie mobile. On peut également transmettre le résultat de l'évaluation par courrier. Le résultat de l'évaluation peut être accompagné, le cas échéant, par la prescription d'un produit ayant une action sur l'état de la peau, son élasticité par exemple.

Le dispositif peut comporter une partie d'acquisition destinée à être appliquée sur différentes régions du corps et notamment sur le bras, l'avant-bras ou la cuisse.

Le cas échéant, des acquisitions peuvent être effectuées alors que le bras est tendu puis relâché, de manière à déterminer l'influence d'une tension de la peau sur les valeurs de module d'Young mesurées, par exemple.

Dans un exemple de mise en oeuvre de l'invention, on mémorise des informations relatives à l'analyse effectuée, par exemple relatives à l'élasticité de la peau, et l'on compare ces valeurs afin par exemple de mettre en évidence une amélioration ou une dégradation dans le temps de l'état de la peau, et notamment de son élasticité.

L'invention est utilisable pour un procédé de prescription d'un produit, notamment un produit cosmétique, ce procédé pouvant comporter les étapes suivantes :

- évaluer une caractéristique mécanique, par exemple l'élasticité de la peau, en mettant en oeuvre le procédé tel que défini plus haut,
- prescrire, au vu du résultat de l'évaluation, un produit ayant un effet sur cette caractéristique.

Par « produit cosmétique », on désigne un produit tel que défini dans la Directive 93/35/CE du Conseil du 14 juin 1993.

L'invention a encore pour objet, selon un autre de ses aspects, un procédé pour déterminer l'efficacité d'un traitement cosmétique ayant une action sur une propriété mécanique de la peau, notamment l'élasticité ou la tension de la peau, comportant les étapes suivantes :

- effectuer une première évaluation de ladite propriété mécanique,
- effectuer le traitement,
- effectuer après le traitement une deuxième évaluation de ladite propriété mécanique, l'une au moins des première et deuxième évaluations étant effectuées en mettant en oeuvre un procédé tel que défini plus haut. De préférence, les deux évaluations sont effectuées en mettant en oeuvre le même procédé.

L'invention a encore pour objet, selon un autre de ses aspects, un procédé de traitement cosmétique d'une région du corps, comportant les étapes suivantes :

- évaluer une propriété mécanique de la peau, notamment l'élasticité ou la tension de la peau dans ladite région, en mettant en oeuvre un procédé tel que défini plus haut,
- effectuer un traitement ayant une action sur ladite propriété mécanique, au vu du résultat de l'évaluation. Ce traitement peut s'effectuer par voie topique, orale ou autre. Le traitement peut comporter le suivi d'un régime alimentaire ou sportif particulier, ou l'administration de soins spécifiques tels que des massages.

L'invention est utilisable pour une méthode pour promouvoir la vente d'un produit, notamment cosmétique, comportant l'étape consistant à faire état d'une activité ou d'une efficacité du produit mise en évidence par un dispositif ou par un procédé tel que défini plus haut.

Une telle promotion du produit pourra se faire par n'importe quel canal de communication.

Elle pourra être faite notamment par un vendeur directement sur le point de vente, par la radio, la télévision ou le téléphone, notamment dans le cadre de spots publicitaires ou de messages courts. Elle pourra être faite également par le canal de la presse écrite ou par le biais de tout autre document, en particulier à des fins publicitaires. Elle pourra se faire également par Internet, par tout autre réseau informatique adéquat ou par un réseau de téléphonie mobile. Elle pourra être faite également directement sur le produit, notamment sur son packaging ou sur toute notice explicative qui lui est associée.

L'invention s'applique à l'analyse de la peau humaine, naturelle, la mesure pouvant avoir lieu *in vivo,* ou la peau artificielle.

L'invention pourra être mieux comprise à la lecture de la description détaillée qui va suivre, d'exemples de mise en oeuvre non limitatifs de celle-ci, et à l'examen du dessin annexé, sur lequel :

- la figure 1 est une vue schématique en élévation avec une coupe axiale partielle d'un dispositif conforme à un exemple de mise en oeuvre de l'invention,
- la figure 2 est une coupe transversale selon II-II de la figure 1,
- la figure 3 représente isolément un dispositif de traitement associé à la partie d'acquisition de la figure 1,
- la figure 4 est un graphe représentant le retard de phase en fonction de la profondeur, et
- la figure 5 est un schéma en blocs illustrant un exemple de procédé selon l'invention.

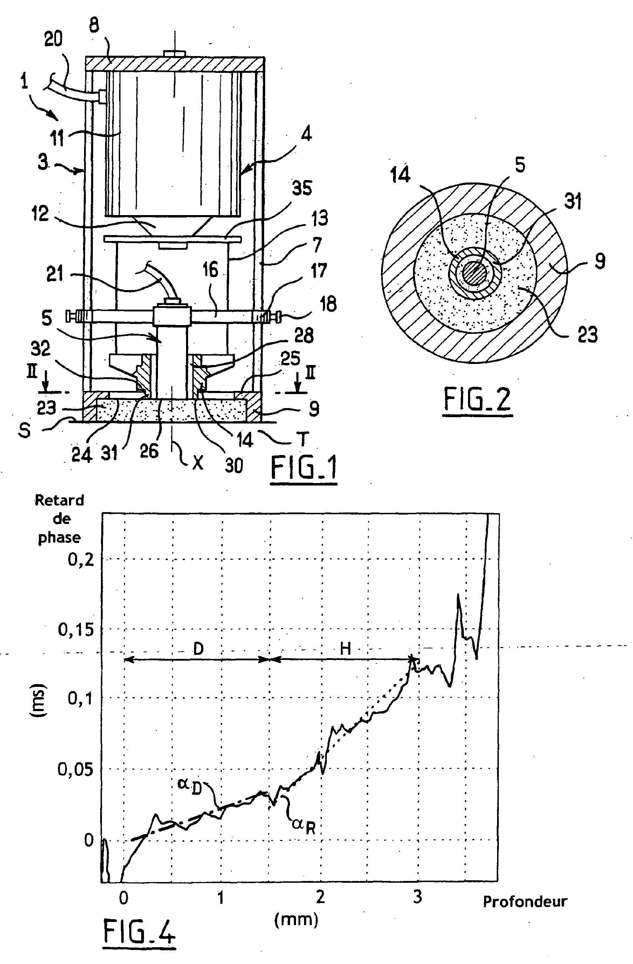

On a représenté à la figure 1 la partie d'acquisition 1 d'un dispositif d'analyse de la peau humaine T, destiné à mesurer la vitesse de propagation d'une onde de cisaillement dans celui-ci.

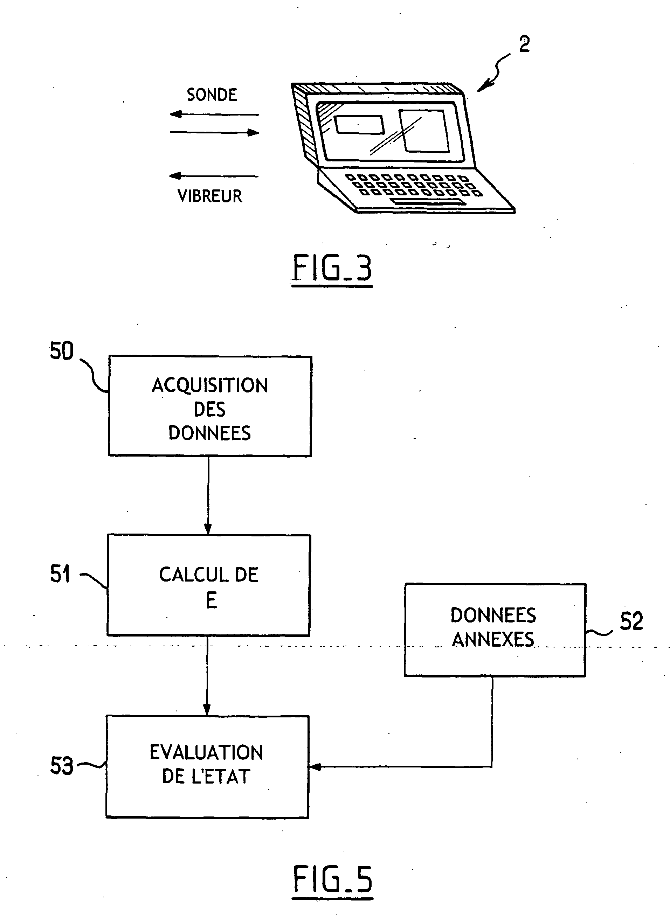

La partie d'acquisition 1 est reliée à un dispositif de traitement 2 que l'on a représenté schématiquement à la figure 3 et qui est constitué par exemple par un micro-ordinateur conventionnel muni de cartes appropriées permettant d'assurer l'interface avec la partie d'acquisition 1.

Cette dernière comporte un châssis 3 supportant un vibreur 4 et une sonde ultrasonore 5, d'axe X.

Plus particulièrement, dans l'exemple considéré, le châssis 3 comporte une pluralité de tiges parallèles 7 fixées à leur extrémité supérieure à une entretoise 8 et à leur extrémité inférieure à un anneau 9 de maintien d'un organe de couplage 23. Les tiges 7 sont au nombre de trois dans l'exemple décrit.

Le vibreur 4 comporte un mécanisme électromagnétique comportant une bobine 11 agencée pour faire vibrer un noyau 12 relié, par l'intermédiaire d'un plateau 35 et de tiges parallèles 13, à une pièce annulaire 14 dont le rôle sera précisé plus loin. Les tiges 13 sont au nombre de trois par exemple.

La sonde 5 est solidaire de bras 16 qui présentent des extrémités 17 qui sont fixées de manière réglable sur les tiges 7 à la hauteur souhaitée grâce à des vis 18. Les bras 16 sont au nombre de trois dans l'exemple décrit.

La bobine 11 et la sonde 5 sont reliées au dispositif de traitement 2 par des câbles respectifs 20 et 21.

L'organe de couplage 23 précité se présente dans l'exemple considéré sous la forme d'un disque d'épaisseur sensiblement constante, réalisé en un gel de gélatine à une concentration de 10 %, ce disque ayant une face inférieure venant reposer contre la surface S du tissu T et une face supérieure 24 venant en appui contre un retour 25 de l'anneau 9.

La surface S du tissu T est perpendiculaire à l'axe X de la sonde.

La sonde 5 vient s'appliquer par une face inférieure 26 contre la face supérieure 24 de l'organe de couplage 23, après avoir traversé la pièce annulaire 14 à la faveur d'une lumière centrale 28.

La pièce annulaire 14 définit une surface de contact 30 avec l'organe de couplage 23 relativement étroite, cette surface de contact 30 étant formée dans l'exemple considéré par la tranche d'extrémité inférieure, perpendiculaire à l'axe X, d'une jupe cylindrique 31 formant un décrochement 32 avec le reste de la pièce 14.

La sonde 5 comporte dans l'exemple considéré un transducteur agencé pour opérer à une fréquence de 50 MHz.

Le dispositif de traitement 2 est agencé pour commander le fonctionnement de la sonde 5 et du vibreur 4 de manière à ce que le vibreur 4 transmette au tissu, par l'intermédiaire de l'organe de couplage 23, une onde de cisaillement. Cette dernière se propage dans la peau à une vitesse qui dépend du module d'Young de celui-ci. La sonde 5 permet de mesurer les déplacements selon l'axe X des différentes couches de la peau sous l'effet de la propagation de cette onde de cisaillement.

En fonction de l'amplitude des déplacements mesurés, on peut déterminer le retard de phase de l'onde de cisaillement à une profondeur donnée et calculer par exemple le module d'Young du derme et de l'hypoderme.

Dans l'exemple considéré, l'onde de cisaillement est générée en excitant le vibreur 4 avec un signal sinusoïdal de fréquence 300 Hz, l'avantage d'une telle fréquence étant de permettre un amortissement rapide de l'onde de cisaillement dans la peau et donc de minimiser les perturbations induites par les échos de l'onde de cisaillement sur les couches plus profondes de la peau.

Un cycle d'acquisition comporte par exemple l'émission en continu d'une onde de cisaillement à la fréquence de 300 Hz et l'acquisition d'images ultrasonores dans l'axe X à des intervalles de temps de 0,5 ms, par exemple, grâce à la sonde 5.

Cette dernière présente dans l'exemple considéré une distance focale de 13 mm, et l'épaisseur de l'organe de couplage 23 est de 12 mm, de telle sorte que les ondes ultrasonores émises par la sonde 5 se focalisent essentiellement dans les premiers millimètres de la peau T à compter de sa surface S.

Chaque image selon l'axe X est par exemple enregistrée sous forme numérique avec une vitesse d'échantillonnage de 500 MHz, l'image comportant par exemple 4096 points le long de l'axe X.

Les images sont ensuite traitées de manière à mettre en évidence des déplacements des différentes couches du tissu, dus à la propagation de l'onde de cisaillement, ce traitement pouvant s'effectuer par exemple par intercorrélation, d'une manière similaire à ce qui a été décrit dans la demande internationale WO 00/55616 précitée.

La figure 4 est un graphe représentant le retard de phase en fonction de la profondeur.

On peut discerner sur ce graphe deux régions D et H correspondant respectivement à la propagation de l'onde de cisaillement dans le derme et dans l'hypoderme.

Les pentes $\alpha_D$ et $\alpha_R$ des droites de régression linéaire dans les régions D et H permettent de connaître le module d'Young E approximatif dans ces couches.

En effet, la vitesse de propagation Vs des ondes de cisaillement correspond aux pentes $\alpha_D$ et $\alpha_R$ et est reliée au coefficient de cisaillement $\mu$ par la relation :

$$Vs = \sqrt{\frac{\mu}{\rho}}$$

où $\rho$ désigne la densité du milieu.

Par ailleurs, dans les tissus biologiques, on peut approcher le module d'Young E par la relation suivante :

$$E \approx 3\,\mu.$$

Le dispositif qui vient d'être décrit peut être utilisé par exemple de la manière suivante.

On procède dans une première étape 50, comme illustré à la figure 5, à l'acquisition des données au moyen de la partie d'acquisition 1, en appliquant par exemple l'anneau 9 et l'organe de couplage 23 sur la cuisse, l'avant-bras ou le bras, puis à l'étape 51 on calcule le module d'Young E pour le derme et l'hypoderme.

En comparant avec des données annexes 52 telles que par exemple des modules d'Young de référence pour un sujet de mêmes sexe et âge que celui objet de l'évaluation, on peut évaluer à l'étape 53 l'état de la peau de ce sujet, notamment lui indiquer si sa peau est plus ou moins élastique que la moyenne et lui prescrire le cas échéant un traitement destiné à améliorer la souplesse de la peau ou à retarder les effets du vieillissement.

Bien entendu, l'invention n'est pas limitée aux exemples qui viennent d'être décrits.

On peut notamment apporter diverses modifications au dispositif qui vient d'être décrit, notamment à la partie d'acquisition destinée à être appliquée sur la peau. En particulier, l'organe de couplage 23 peut être réalisé par exemple avec une forme autre et en particulier dans un matériau autre que de la gélatine, ce qui peut permettre le cas échéant de réaliser des ajours ou évidements dans l'organe de couplage, destinés à améliorer le découplage mécanique entre la sonde ultrasonore ou le vibreur et/ou à améliorer la forme des ondes transmises à la peau.

On peut réaliser la pièce du vibreur destinée à transmettre l'onde de cisaillement à la peau avec une forme non symétrique de révolution, notamment si l'on souhaite mettre en évidence une anisotropie des fibres de collagène.

Dans toute la description, y compris les revendications, l'expression « comportant un » doit être comprise comme étant synonyme de « comportant au moins un », sauf si le contraire est spécifié.

**Revendications**

1.    Dispositif d'analyse de la peau (T), comportant :

- une sonde ultrasonore (5) agencée pour analyser la peau (T) selon un axe (X),
- un vibreur (4) agencé pour transmettre à une région de la peau (T) s'étendant autour de l'axe (X) au moins une onde de cisaillement à partir de ladite région, la sonde ultrasonore (5) étant agencée pour détecter des déplacements induits dans la peau par la propagation de l'onde de cisaillement.

2.    Dispositif selon la revendication précédente, **caractérisé par le fait qu'**il comporte un organe de couplage (23) permettant la transmission d'ondes ultrasonores entre la sonde (5) et la peau (T).

3.    Dispositif selon la revendication précédente, **caractérisé par le fait que** l'épaisseur de l'organe de couplage (23) est choisie de manière à permettre la focalisation des ondes ultrasonores dans une région de profondeur maximale donnée à partir de la surface (S) de la peau (T), notamment une région de profondeur inférieure ou égale à 4 mm.

4.    Dispositif selon l'une quelconque des revendications précédentes, **caractérisé par le fait que** la distance focale de la sonde ultrasonore (5) est comprise entre 10,4 mm et 15,6 mm.

5.    Dispositif selon l'une des revendications 2 ou 3, **caractérisé par le fait que** l'épaisseur de l'organe de couplage (23) est comprise entre 10,6 mm et 14,4 mm.

6.    Dispositif selon l'une quelconque des revendications 2, 3 ou 5, **caractérisé par le fait que** l'organe de couplage (23) se présente sous la forme d'un disque d'un matériau viscoélastique.

**7.** Dispositif selon la revendication 6, **caractérisé par le fait que** l'organe de couplage (23) est maintenu contre la surface (S) de la peau (T) par un anneau de maintien (9) pourvu d'un retour vers l'intérieur (25), contre lequel une face de l'organe de couplage (23) opposée à la peau (T) peut prendre appui.

**8.** Dispositif selon la revendication précédente, **caractérisé par le fait qu'**il comporte un châssis (3) dont sont solidaires le vibreur (4) et 1a sonde (5), le châssis étant réalisé de manière à permettre de positionner le dispositif de telle manière que l'axe (X) soit sensiblement perpendiculaire à la surface (S) de la peau (T).

**9.** Dispositif selon l'une quelconque des revendications précédentes, **caractérisé par le fait que** ledit vibreur (4) comporte une pièce annulaire (14) définissant une surface de contact (30) à partir de laquelle l'onde de cisaillement est émise vers la peau, cette pièce annulaire présentant une lumière centrale (28) dans laquelle s'étend la sonde ultrasonore (5).

**10.** Dispositif selon la revendication précédente, **caractérisé par le fait que** la surface de contact (30) présente une symétrie par rapport à l'axe (X), notamment une symétrie de révolution autour de l'axe (X).

**11.** Dispositif selon l'une quelconque des revendications précédentes, **caractérisé par le fait que** la sonde (5) est agencée pour transmettre et recevoir les ondes ultrasonores à une fréquence comprise entre 1 et 300 MHz, mieux entre 30 et 70 MHz, mieux encore de 50 MHz.

**12.** Dispositif selon l'une quelconque des revendications précédentes, **caractérisé par le fait qu'**il comporte un générateur agencé pour alimenter le vibreur (4) avec un signal basse fréquence pendant toute la durée de l'analyse, le signal ayant une fréquence comprise entre 100 et 500 Hz, étant de préférence de 300 Hz environ.

**13.** Dispositif selon l'une quelconque des revendications précédentes, **caractérisé par le fait qu'**il comporte un dispositif de traitement (2) agencé pour délivrer au moins une information représentative d'une propriété mécanique et/ou de l'épaisseur d'une couche au moins de la peau (T) à partir des signaux recueillis par la sonde ultrasonore (5).

**14.** Dispositif selon la revendication précédente, **caractérisé par le fait que** le dispositif de traitement est agencé pour délivrer une information relative à l'état de la peau, notamment son état de vieillissement, à partir de 1a comparaison d'une valeur mesurée avec une valeur de référence.

**15.** Dispositif selon l'une des revendications 13 et 14, **caractérisé par le fait que** le dispositif de traitement est agencé pour effectuer une mémorisation des signaux recueillis par la sonde ultrasonore à différents instants successifs, notamment tous les intervalles de temps *dt* pendant *n* fois, *dt* étant compris par exemple entre 0,2 et 0,8 ms et *n* compris entre 50 et 500.

**16.** Dispositif selon l'une quelconque des revendications précédentes, **caractérisé par le fait que** la sonde (5) et le vibreur (4) sont agencés de telle sorte que le déplacement du vibreur permettant de générer l'onde de cisaillement n'est pas transmis à la sonde.

**17.** Procédé d'analyse de la peau, **caractérisé par le fait qu'**il comporte l'étape consistant à analyser la peau (T) au moyen d'un dispositif selon l'une quelconque des revendications précédentes.

**18.** Procédé selon la revendication précédente, **caractérisé par le fait qu'**il comporte en outre l'étape de traitement des signaux provenant de la sonde ultrasonore (5) afin de déterminer au moins une valeur liée à des propriétés mécaniques de la peau, notamment le module d'Young (E), le module de cisaillement ($\mu$) ou la vitesse de propagation (Vs) de l'onde de cisaillement.

**19.** Procédé selon l'une des revendications 17 et 18, **caractérisé par le fait que** l'on calcule le retard de phase de l'onde de cisaillement en fonction de la profondeur.

**20.** Procédé selon l'une quelconque des revendications 17 à 19, **caractérisé par le fait que** l'on détermine un état de la peau, notamment un état de vieillissement de la peau, à partir de la comparaison d'une valeur de module d'Young provenant de l'analyse de la peau avec des valeurs de référence.

**21.** Procédé pour évaluer une propriété mécanique, notamment l'élasticité, d'une région de la peau, comportant les étapes suivantes :

    - analyser ladite région au moyen du dispositif selon l'une quelconque des revendications 1 à 16,
    - délivrer à partir des résultats de l'analyse une information relative à ladite propriété mécanique.

**22.** Procédé pour déterminer l'efficacité d'un traitement cosmétique ayant une action sur une propriété mécanique de la peau, notamment son élasticité ou sa tension, comportant les étapes suivantes :

    - effectuer une première évaluation de ladite propriété mécanique,

- effectuer le traitement,

- effectuer après le traitement une deuxième évaluation de ladite propriété mécanique, l'une au moins des première et deuxième évaluations étant effectuées en mettant en oeuvre le procédé selon la revendication 21.

23. Procédé de traitement cosmétique d'une région du corps, comportant les étapes suivantes :

- évaluer une propriété mécanique de la peau dans ladite région en mettant en oeuvre le procédé selon la revendication 21,
- effectuer un traitement cosmétique ayant une action sur ladite propriété, au vu du résultat de l'évaluation.

**Claims**

1. Analysis apparatus for analyzing the skin (T), the apparatus comprising:

   · an ultrasonic probe (5) arranged to analyze the skin (T) along an axis (X); and
   · a vibrator (4) arranged to emit at least one shear wave to a region of the skin (T) extending about the axis (X) from said region, the ultrasonic probe (5) being arranged to detect displacements induced in the skin by the propagation of the shear wave.

2. Apparatus according to the preceding claim, **characterized by** the fact that it includes a coupling member (23) enabling ultrasonic waves to be transmitted between the probe (5) and the skin (T).

3. Apparatus according to the preceding claim, **characterized by** the fact that a thickness of the coupling member (23) is selected so as to enable the ultrasonic waves to be focused in a given region of maximum depth below a surface (S) of the skin (T), in particular a region being less than or equal to 4 mm in depth.

4. Apparatus according to any preceding claim, **characterized by** the fact that a focal length of the ultrasonic probe (5) lies in the range 10.4 mm to 15.6 mm.

5. Apparatus according to claim 2 or claim 3, **characterized by** the fact that the thickness of the coupling member (23) lies in the range 10.6 mm to 14.4 mm.

6. Apparatus according to claim 2, claim 3, or claim 5, **characterized by** the fact that the coupling member (23) is in the form of a disk of viscoelastic material.

7. Apparatus according to claim 6, **characterized by** the fact that the coupling member (23) is held against the surface (S) of the skin (T) by a holding ring (9) provided with an inwardly-directed rim (25) against which a face of the coupling member (23) opposed to the skin (T) can bear.

8. Apparatus according to the preceding claim, **characterized by** the fact that it includes a frame (3) to which the vibrator (4) and the probe (5) are secured, the frame being made so as to enable the apparatus to be positioned in such a manner that the axis (X) is substantially perpendicular to a surface (S) of the skin (T).

9. Apparatus according to any preceding claim, **characterized by** the fact that said vibrator (4) includes an annular piece (14) defining a contact surface (30) from which the shear wave is emitted to the skin, the annular piece presenting a central bore (28) in which the ultrasonic probe (5) extends.

10. Apparatus according to the preceding claim, **characterized by** the fact that the contact surface (30) presents symmetry about the axis (X), in particular a circular symmetry about the axis (X).

11. Apparatus according to any preceding claim, **characterized by** the fact that the probe (5) is arranged to emit and receive ultrasonic waves at a frequency lying in the range 1 MHz to 300 MHz, preferably in the range 30 MHz to 70 MHZ, and more preferably at a frequency of 50 MHz.

12. Apparatus according to any preceding claim, **characterized by** the fact that it includes a generator arranged to deliver a low--frequency signal to the vibrator (4) during the entire analysis period, the signal having a frequency lying in the range 100 Hz to 500 Hz, preferably being of about 300 Hz.

13. Apparatus according to any preceding claim, **characterized by** the fact that it includes a processor device (2) arranged to deliver at least one piece of information, representative of a mechanical property and/or of a thickness of at least one layer of the skin (T), from signals picked up by the ultrasonic probe (5).

14. Apparatus according to the preceding claim, **characterized by** the fact that the processor device is arranged to deliver a piece of information relating to the state of the skin, in particular its state of aging, by comparing a measured value with a reference value.

15. Apparatus according to claim 13 or claim 14, **characterized by** the fact that the treatment device is arranged to store the signals picked up by the ultrasonic probe at various successive instants, in particular at all n time intervals dt, n lying in the range 50 to 500, and dt lying in the range 0.2 ms to 0.8 ms.

16. Apparatus according to any preceding claim, **characterized by** the fact that the probe (5) and the vibrator (4) are arranged so that the displacement of the vibrator for generating the shear wave is not transmitted to the probe.

17. A skin analysis method, **characterized by** the fact that it comprises the step consisting in analyzing the skin (T) by means of apparatus according to any preceding claim.

18. A method according to the preceding claim, **characterized by** the fact that it further comprises the step of processing signals coming from the ultrasonic probe (5) so as to determine at least one value relating to mechanical properties of the skin, in particular the Young's modulus (E), the shear modulus ($\mu$) or the propagation speed (Vs) of the shear wave.

19. A method according to claim 17 or claim 18, **characterized by** the fact that the phase lag of the shear wave is calculated as a function of the depth.

20. A method according to any one of claims 17 to 19, **characterized by** the fact that a state of the skin, in particular a state of aging of the skin, is determined by comparing a value for Young's modulus resulting from analyzing the skin with reference values.

21. A method of evaluating a mechanical property, in particular the elasticity, of a region of the skin, the method comprising:

· analyzing said region with the apparatus according to any one of claims 1 to 16; and
· delivering, from the results of the analysis, a piece of information relating to said mechanical property.

22. A method of determining the effectiveness of cosmetic treatment that has action on a mechanical property of the skin, in particular its elasticity or its tension, the method comprising:

performing a first evaluation of said mechanical property;
· performing the treatment; and
· after the treatment, performing a second evaluation of said mechanical property, at least one of the first and second evaluations being performed by implementing the method according to claim 21.

23. A method of cosmetic treatment of a region of the body, the method comprising:

· evaluating a mechanical property of the skin in said region by implementing the method according to claim 21; and
· performing a cosmetic treatment that has action on said property in the light of the result of the evaluation.

**Patentansprüche**

1. Vorrichtung zur Analyse der Haut (T), umfassend:

- eine Ultraschallsonde (5), die dafür ausgebildet ist, die Haut (T) in einer Achse (X) zu analysieren,
- einen Vibrator (4), der dafür ausgebildet ist, auf einen sich um die Achse (X) herum erstreckenden Bereich der Haut (T) mindestens eine Scherwelle ausgehend von diesem Bereich zu übertragen, wobei die Ultraschallsonde (5) dafür ausgebildet ist, Bewegungen zu erfassen, die in der Haut durch die Ausbreitung der Scherwelle induziert werden.

2. Vorrichtung nach dem vorhergehenden Anspruch, **dadurch gekennzeichnet, dass** sie ein Kopplungsorgan (23) umfasst, das die Übertragung von Ultraschallwellen zwischen der Sonde (5) und der Haut (T) gestattet.

3. Vorrichtung nach dem vorhergehenden Anspruch, **dadurch gekennzeichnet, dass** die Dicke des Kopplungsorgans (23) so gewählt ist, dass sie die Fokussierung der Ultraschallwellen in einem gegebenen maximalen Tiefenbereich von der Oberfläche (S) der Haut (T) aus, insbesondere in einem Tiefenbereich kleiner als oder gleich 4 mm, gestattet.

4. Vorrichtung nach einem der vorhergehenden Ansprüche, **dadurch gekennzeichnet, dass** die Brennweite der Ultraschallsonde (5) zwischen 10,4 mm und 15,6 mm beträgt.

5. Vorrichtung nach einem der Ansprüche 2 oder 3, **dadurch gekennzeichnet, dass** die Dicke des Kopplungsorgans (23) zwischen 10,6 mm und 14,4 mm beträgt.

6. Vorrichtung nach einem der Ansprüche 2, 3 oder 5, **dadurch gekennzeichnet, dass** das Kopplungsorgan (23) in der Form einer Scheibe aus einem viskoelastischen Werkstoff vorliegt.

7. Vorrichtung nach Anspruch 6, **dadurch gekennzeichnet, dass** das Kopplungsorgan (23) an der Oberfläche (S) der Haut (T) durch einen Haltering (9) gehalten wird, der mit einer Abwinklung (25) nach Innen versehen ist, an der eine der Haut (T) entgegengesetzte Seite des Kopplungsorgans (23) anliegen kann.

**8.** Vorrichtung nach dem vorhergehenden Anspruch, **dadurch gekennzeichnet, dass** sie ein Gestell (3) umfasst, mit dem der Vibrator (4) und die Sonde (5) fest verbunden sind, wobei das Gestell so ausgebildet ist, dass es gestattet, die Vorrichtung so zu positionieren, dass die Achse (X) zur Oberfläche (S) der Haut (T) im Wesentlichen senkrecht ist.

**9.** Vorrichtung nach einem der vorhergehenden Ansprüche, **dadurch gekennzeichnet, dass** der Vibrator (4) ein ringförmiges Teil (14) umfasst, das eine Kontaktfläche (30) bildet, von der aus die Scherwelle auf die Haut zu gesendet wird, wobei dieses ringförmige Teil eine zentrale Öffnung (28) aufweist, in der sich die Ultraschallsonde (5) erstreckt.

**10.** Vorrichtung nach dem vorhergehenden Anspruch, **dadurch gekennzeichnet, dass** die Kontaktfläche (30) eine Symmetrie bezüglich der Achse (X), insbesondere eine Rotationssymmetrie um die Achse (X), besitzt.

**11.** Vorrichtung nach einem der vorhergehenden Ansprüche, **dadurch gekennzeichnet, dass** die Sonde (5) ausgebildet ist, um die Ultraschallwellen mit einer Frequenz zwischen 1 und 300 MHz, besser zwischen 30 und 70 MHz, noch besser von 50 MHz, zu übertragen und zu empfangen.

**12.** Vorrichtung nach einem der vorhergehenden Ansprüche, **dadurch gekennzeichnet, dass** sie einen Generator umfasst, der ausgebildet ist, um den Vibrator (4) während der gesamten Dauer der Analyse mit einem Niederfrequenzsignal zu versorgen, wobei das Signal eine Frequenz zwischen 100 und 500 Hz, vorzugsweise etwas 300 Hz, besitzt.

**13.** Vorrichtung nach einem der vorhergehenden Ansprüche, **dadurch gekennzeichnet, dass** sie eine Verarbeitungsvorrichtung (2) umfasst, die ausgebildet ist, um mindestens eine Information, die für eine mechanische Eigenschaft und/oder die Dicke mindestens einer Schicht der Haut (T) repräsentativ ist, ausgehend von den von der Ultraschallsonde (5) aufgenommenen Signalen zu liefern.

**14.** Vorrichtung nach dem vorhergehenden Anspruch, **dadurch gekennzeichnet, dass** die Verarbeitungsvorrichtung ausgebildet ist, um eine den Zustand der Haut, insbesondere ihren Alterungszustand, betreffende Information ausgehend von dem Vergleich eines gemessenen Werts mit einem Bezugswert zu liefern.

**15.** Vorrichtung nach einem der Ansprüche 13 und 14, **dadurch gekennzeichnet, dass** die Verarbeitungsvorrichtung ausgebildet ist, um eine Speicherung der Signale vorzunehmen, die von der Ultraschallsonde zu verschiedenen aufeinanderfolgenden Zeitpunkt aufgenommen werden, insbesondere alle Zeitintervalle $dt$ während $n$ Malen, wobei $dt$ beispielsweise zwischen 0,2 und 0,8 ms und $n$ zwischen 50 und 500 beträgt.

**16.** Vorrichtung nach einem der vorhergehenden Ansprüche, **dadurch gekennzeichnet, dass** die Sonde (5) und der Vibrator (4) so ausgebildet sind, dass die Bewegung des Vibrators, die die Erzeugung der Scherwelle gestattet, nicht auf die Sonde übertragen wird.

**17.** Verfahren zur Analyse der Haut, **dadurch gekennzeichnet, dass** es den Schritt umfasst, der darin besteht, dass die Haut (T) mit Hilfe einer Vorrichtung nach einem der vorhergehenden Ansprüche analysiert wird.

**18.** Verfahren nach dem vorhergehenden Anspruch, **dadurch gekennzeichnet, dass** es außerdem den Schritt der Verarbeitung der von der Ultraschallsonde (5) kommenden Signale umfasst, um mindestens einen mit mechanischen Eigenschaften der Haut verbundenen Wert zu bestimmen, insbesondere den Youngmodul (E), den Schermodul ($\mu$) oder die Fortpflanzungsgeschwindigkeit (Vs) der Scherwelle.

**19.** Verfahren nach einem der Ansprüche 17 und 18, **dadurch gekennzeichnet, dass** man die Phasenverzögerung der Scherwelle in Abhängigkeit von der Tiefe berechnet.

**20.** Verfahren nach einem der Ansprüche 17 bis 19, **dadurch gekennzeichnet, dass** man den Zustand der Haut, insbesondere einen Alterungszustand der Haut, ausgehend von dem Vergleich eines von der Analyse der Haut stammenden Youngmodulwerts mit Bezugswerten bestimmt.

**21.** Verfahren zum Messen einer mechanischen Eigenschaft, insbesondere der Elastizität, eines Bereichs der Haut, umfassend die folgenden Schritte:

- diesen Bereich mit Hilfe der Vorrichtung nach einem der Ansprüche 1 bis 16 analysieren,
- ausgehend von den Ergebnissen der Analyse eine Information über diese mechanische Eigenschaft liefern.

**22.** Verfahren zur Bestimmung der Wirksamkeit einer kosmetischen Behandlung, die eine Wirkung auf eine mechanische Eigenschaft der Haut, insbesondere auf ihre Elastizität oder ihre Spannung, hat, umfassend die folgenden Schritte:

- eine erste Messung dieser mechanischen Eigenschaft vornehmen,
- die Behandlung vornehmen,
- nach der Behandlung eine zweite Messung der mechanischen Eigenschaft vornehmen, wobei mindestens eine der ersten und der zweiten Messung unter Anwendung des Verfahrens nach Anspruch 21 durchgeführt wird.

23. Verfahren zur kosmetischen Behandlung eines Bereichs des Körpers, umfassend die folgenden Schritte:

- eine mechanische Eigenschaft in diesem Bereich unter Anwendung des Verfahrens nach Anspruch 21 messen,
- eine kosmetische Behandlung, die eine Wirkung auf diese Eigenschaft hat, unter Berücksichtung des Ergebnisses der Messung vornehmen.

FIG.1

FIG.2

Retard de phase

(ms)

Profondeur (mm)

FIG.4

SONDE

VIBREUR

FIG.3

50 — ACQUISITION DES DONNEES

51 — CALCUL DE E

52 — DONNEES ANNEXES

53 — EVALUATION DE L'ETAT

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0055616 A
- FR 2655835